# EUROPÄISCHE PATENTSCHRIFT

(11) **EP 1 635 905 B1**
(45) Veröffentlichungstag und Bekanntmachung des Hinweises auf die Patenterteilung: **30.11.2011**
(21) Anmeldenummer: 04739550.4
(22) Anmeldetag: 03.06.2004
(51) Int. Cl.: A61N 1/36

(54) **AKTIVES RETINA-IMPLANTAT MIT EINER VIELZAHL VON BILDELEMENTEN**
ACTIVE RETINA IMPLANT COMPRISING A PLURALITY OF PICTURE ELEMENTS
IMPLANT RETINIEN ACTIF COMPRENANT UNE PLURALITE D'ELEMENTS D'IMAGE

(30) Priorität: 23.06.2003 DE 10329615
(43) Veröffentlichungstag der Anmeldung: 22.03.2006
(60) Teilanmeldung: 09161695.3
(73) Patentinhaber: Retina Implant AG, 72770 Reutlingen (DE)
(72) Erfinder: GRAF, Heinz, Gerhard, 71106 Magstadt (DE); DOLLBERG, Alexander, 44225 Dortmund (DE); HÖFFLINGEN, Bernd, 71063 Sindelfingen (DE); NISCH, Wilfried, 72070 Tübingen (DE); HÄMMERLE, Hugo, 72072 Tübingen (DE); STETT, Alfred, 72770 Reutlingen (DE); STELZLE, Martin, 72776 Reutlingen (DE); ZRENNER, Eberhart, 72076 Tübingen (DE)
(74) Vertreter: Otten, Hajo
(86) Internationale Anmeldenummer: PCT/EP2004/005975
(87) Internationale Veröffentlichungsnummer: WO 2005/000395

(56) Entgegenhaltungen:
- EP-A- 0 460 320
- DE-A- 19 921 399
- US-A- 5 895 415

## Beschreibung

Die vorliegende Erfindung betrifft ein aktives Retina-Implantat mit einer Vielzahl von Bildelementen, die einfallendes Licht in elektrische Stimulationssignale für über Stimulationselektroden zu kontaktierende Zellen der Retina umwandeln, wobei jedes Bildelement zumindest eine Bildzelle, die einfallendes Licht in elektrische Signale umwandelt, sowie zumindest einen Verstärker aufweist, dessen Eingang mit der Bildzelle und dessen Ausgang mit zumindest einer Stimulationselektrode verbunden ist, an die er ein Stimulationssignal liefert, und mit einer Energieversorgung, die extern angekoppelte Fremdenergie als Versorgungsspannung für die Bildzellen und die Verstärker bereitstellt.

Ein derartiges Retina-Implantat ist beispielsweise aus der DE 197 05 988 A1 bekannt.

Ein derartiges Retina-Implantat ist beispielsweise aus der DE 197 05 988 A1 bekannt.

Das bekannte Retina-Implantat dient dazu, einem Verlust des Sehvermögens auf Grund von Retina-Degenerationen entgegenzuwirken. Grundgedanke ist es dabei, einem Patienten einen mikroelektronischen Stimulationschip im Bereich der degenerierten Retina zu implantieren, der die Funktion beispielsweise von degenerierten Fotorezeptoren ersetzen soll. Der Stimulationschip besitzt eine Vielzahl von Bildelementen, die in Abhängigkeit von dem einfallenden sichtbaren Licht elektrische Impulse im Bereich der Retina erzeugen und dabei Zellen in der Retina stimulieren.

Das Retina-Implantat kann als epiretinales Implantat auf die Retina aufgesetzt sein, oder aber es kann in bzw. unter der Retina als so genanntes subretinales Implantat eingesetzt sein.

Ein subretinales Implantat ist beispielsweise aus der EP 0 460 320 A2 bekannt. Bei diesem Implantat soll das auftreffende Umgebungslicht ausreichen soll, um die erforderlichen Stimuli für die Zellen in der Retina zu erzeugen. Bezüglich der genauen Platzierung eines subretinalen Retina-Implantates wird auf dieses Dokument verwiesen.

In der eingangs genannten DE 197 05 988 A1 ist ein subretinales Implantat beschrieben, das mit einer für nicht-sichtbare elektromagnetische Strahlung wirksamen, fotovoltaischen Schicht versehen ist, wobei die Stimulationssignale unter Ausnutzung der von der fotovoltaischen Schicht erzeugten Spannung lokal geschaltet werden. Dem bekannten Implantat liegt die Idee zu Grunde, mit Hilfe von elektromagnetischer Strahlung aus dem nicht-sichtbaren Spektralbereich, nämlich Infrarotstrahlung, eine Fremdenergie für den Stimulationschip bereitzustellen. Die fotovoltaische Schicht wirkt dabei wie eine Art Verstärker für die durch das einfallende sichtbare Licht erzeugten Signale. In Folge dessen können auch bei schwachen Lichtverhältnissen im sichtbaren Spektralbereich hinreichend starke Stimulationssignale erzeugt werden.

Bei dem bekannten Retina-Implantat ergibt sich jedoch das Problem, das einfallende sichtbare Licht über einen großen Intensitätsbereich, der bei natürlichen Lichtverhältnissen mehrere Zehnerpotenzen umfasst, in entsprechende elektrische Stimulationssignale umzusetzen.

Vor diesem Hintergrund beschreibt die DE 199 21 399 A1 ein Retina-Implantat, bei dem mindestens ein Bildelement vorgesehen ist, das als Referenzelement wirkt, wobei der Verstärker die Differenz zwischen den Ausgangssignalen des Referenzelementes und der Bildzelle bildet, die die lokale Helligkeit erfasst. Auf diese Weise soll das so erzeugte Stimulationssignal an die Umgebungshelligkeit angepasst werden.

Mit der Problematik der Übertragung von Stimulationssignalen über die Stimulationselektroden auf zu kontaktierende Zellen der Retina beschäftigt sich der Artikel Stelzle et al.: "Electrical Properties of Micro-Photodiode Arrays for Use as Artificial Retina Implant", Biomedical Micro Devices 3:2, 133-142, 2001. Die Autoren berichten, dass die Kopplung zwischen der Stimulationselektrode und dem Gewebe kapazitiver Natur ist, so dass zur Stimulation nur transiente Signale verwendet werden können. Diese kapazitive Kopplung beruht darauf, dass sich an der Grenzfläche zwischen Elektrode und Elektrolyt im Auge eine Kapazität (Helmholz-Doppelschicht) in Folge der Elektrodenpolarisation ausbildet. Die Autoren zeigen für ein passives Implantat, also ein Implantat, wie es in der eingangs erwähnten EP 0 460 320 A2 beschrieben ist, dass ein Pulsen des sichtbaren Nutzlichtes zu einem Grenzzyklus führt, bei dem ein ausgeglichener Ladungstransport in die Kapazität hinein und aus dieser wieder heraus erfolgt. Um die mit dem passiven Implantat verbundenen Probleme zu lösen, schlagen die Autoren vor, Lichtpulse mit einer bestimmten Pulsrate zu verwenden. Ferner wird als wünschenswert angesprochen, eine externe Energieversorgung zu verwenden, um den Stimulationsstrom zu erzeugen. Weiter empfehlen sie die Verwendung einer aktiven Stromsenke, um die mittlere Elektrodenpolarisation zu verringern. Allerdings erwähnen sie, dass wegen der gepulsten Anregung eine vollständige Entladung der Elektrodenkapazität wohl nicht erreichbar ist.

Vor diesem Hintergrund liegt der vorliegenden Erfindung die Aufgabe zu Grunde, das eingangs erwähnte, aktive Retina-Implantat mit schaltungstechnisch einfachen Mitteln derart weiterzubilden, dass eine effektive Umsetzung des einfallenden Lichtes in die Stimulationssignale erreicht wird, damit eine effektive Anregung der Zellen in der Retina auch bei unterschiedlicher Umgebungsbeleuchtung möglich wird.

Bei dem eingangs genannten Retina-Implantat wird diese Aufgabe erfindungsgemäß dadurch gelöst, dass die Bildzelle eine logarithmische Kennlinie aufweist, nach der einfallendes Licht bestimmter Intensität in elektrische Signale bestimmter Amplitude umgewandelt wird.

Die der Erfindung zu Grunde liegende Aufgabe wird auf diese Weise vollkommen gelöst.

Die logarithmische Bildzelle, die als solche bspw. aus der DE 42 09 536 AI seit vielen Jahren bekannt ist, hat ein logarithmisches Verstärkerverhalten, das nach Erkenntnis der Erfinder der Lichtempfindlichkeit des Auges ähnlich ist und daher besonders effizient als Bildzelle verwendet werden kann. Auf diese Weise kann auch ohne Referenzelement und Referenzverstärker ein großer Helligkeitsbereich in Stimulationssignale umgewandelt werden, so dass sowohl bei geringer als auch bei hoher Umgebungsbeleuchtung Sehen mit hinreichendem Kontrast möglich ist.

Weiterhin ist bevorzugt, wenn das Stimulationssignal in Form von analogen Spannungspulsen bestimmter Pulslänge und Pulsabstände geliefert wird, deren Amplitude von der Intensität des einfallenden Lichtes abhängt.

Die Erfinder der vorliegenden Anmeldung haben nämlich erkannt, dass es auch möglich ist, über die Stimulationselektroden analoge Spannungspulse an die Zellen in der Retina abzugeben, wenn eine hinreichende Stimulation der Zellen erreicht werden soll. Die Erfinder gehen damit gerade nicht den üblichen Weg, Strompulse mit Ladungskompensation zu verwenden, sondern verwenden eine Spannungssteuerung, obwohl dies auf Grund eines Vorurteils im Stand der Technik wegen der Elektrodenpolarisation Probleme bereiten sollte.

Die Erfinder haben jedoch erkannt, dass die Spannungssteuerung schaltungstechnisch sehr einfach zu realisieren ist und bei richtiger Auslegung dennoch die Probleme mit der Elektrodenpolarisation vermieden werden können.

Wegen des geringen schaltungstechnischen Aufwandes lässt sich hier nämlich die Dichte der Bildelemente und damit der Stimulationselektroden deutlich erhöhen, so dass hier insgesamt eine bessere örtliche Auflösung erreicht wird, als dies mit den aufwendigen Schaltungen aus dem Stand der Technik möglich ist. Diese höhere Dichte führt zu einer effektiveren Stimulation der Zellen in der Retina, da das lokale Verhältnis zwischen der Zahl der Stimulationselektroden und der Zahl der anzure- - genden Zellen größer wird.

Dabei ist es besonders bevorzugt, wenn der Ausgang des Verstärkers mit einer ansteuerbaren Entladungsschaltung verbunden ist.

Über diese Entladungsschaltung kann eine sich aufbauende Elektrodenpolarisation gezielt wieder abgebaut werden, so dass verhindert wird, dass die Ladung und damit die Spannung in der Grenzflächenkapazität immer mehr zunimmt, was im Gegenzug dazu führen würde, dass das Stimulationssignal immer schlechter auf die Zellen in der Retina übertragen werden könnte.

Dabei ist es bevorzugt, wenn Pulslänge und Pulsabstand über die extern eingekoppelte Fremdenergie bestimmt werden.

Bei dieser Maßnahme ist von Vorteil, dass Pulslänge und Pulsabstand, also Pulsrate, nicht über ein mechanisches Zerhacken des sichtbaren Nutzlichtes wie im Stand der Technik oder über auf dem Retina-Implantat vorzusehende Frequenzgeneratoren erzeugt werden müssen. Der schaltungstechnische Aufwand des Retina-Implantates wird auch auf diese Weise deutlich gering gehalten.

Ein weiterer Vorteil besteht darin, dass Pulslänge und Pulsabstand nach dem Einbringen des Retina-Implantates individuell an die physiologischen Bedingungen bei dem jeweiligen Patienten angepasst werden können. Ferner ist es möglich, Pulslänge und Pulsabstand in Abhängigkeit von den jeweiligen Lichtverhältnissen zu verändern. Mit anderen Worten, durch diese Maßnahme wird über die externe Energieversorgung gleichzeitig auch eine Steuerung der Funktionsweise des Implantates bewirkt.

Die externe Energieversorgung kann dabei beispielsweise eingekoppeltes IR-Licht oder aber induktiv eingekoppelte Energie beispielsweise im HF-Bereich sein.

Weiter ist es bevorzugt, wenn die Entladungsschaltung am Ende eines Spannungspulses derart angesteuert wird, dass der Ausgang des Verstärkers mit einem Entladepotenzial verbunden wird.

Hier ist von Vorteil, dass automatisch am Ende eines jeden stimulierenden Spannungspulses die Elektrodenpolarisierung zurückgeführt wird, da die Kapazität zwischen Stimulationselektrode und umgebendem Gewebe entladen wird. Jeder neue Spannungspuls trifft damit auf einen vollständig entladenen Kondensator, so dass zu Beginn des Spannungspulses ein hoher Stimulationsstrom durch den Kondensator in das Gewebe und zu den Zellen der Retina gelangen kann. Dieser Strom nimmt auf Grund der sich aufbauenden Spannung in dem Grenzflächenkondensator über der Zeit ab. Nach dem Ausschalten des Spannungspulses wird der Ausgang des Verstärkers dann mit dem Entladepotenzial verbunden, das in der Regel die elektrische Masse des Retina-Implantates ist. Dadurch ergibt sich ein großer Entladestrom, der die Grenzschichtkapazität vollständig wieder entlädt.

Es hat sich herausgestellt, dass eine Pulslänge von ca. 500 µs ausreichend ist, um die Zellen in der Retina hinreichend zu stimulieren. Über die Amplitude des Spannungspulses wird dabei die anfängliche Stärke des Einschaltstromes bestimmt.

Der Pulsabstand beträgt dabei vorzugsweise 50 ms, denn eine Wiederholfrequenz von 20 Hz hat sich als ausreichend für flimmerfreies Sehen herausgestellt. Dieser Pulsabstand ist ferner ausreichend, um die Elektrodenpolarisation vollständig zurückzuführen.

Allgemein ist es dabei bevorzugt, wenn die Bildzellen mit einer ersten Spannung versorgt werden, die von einer zweiten Spannung verschieden ist, mit der die Verstärker versorgt werden, wobei die zweite Spannung vorzugsweise mit der Pulslänge und dem Pulsabstand eingeschaltet wird.

Hier ist von Vorteil, dass die Bildzellen beispielsweise ständig mit der ersten Spannung versorgt werden, so dass die elektrischen Signale der Bildzellen ständig zur Verfügung stehen und keine Einschwingvorgänge abgewartet werden müssen. Andererseits werden die Verstärker mit der zweite Spannung versorgt, die mit der Pulslänge und dem Pulsabstand eingeschaltet wird. Auf diese Weise werden die elektrischen Signale der Bildzellen über den getakteten Verstärker in die Spannungspulse umgewandelt. Dies ist schaltungstechnisch besonders einfach, weil es nicht erforderlich ist, eine zusätzliche Taktstufe an den Ausgang des Verstärkers zu schalten, der Verstärker wird vielmehr im Takt der Spannungspulse ein- und dann wieder ausgeschaltet. Da das zeitliche Ein- und Ausschalten der zweiten Spannung von der extern eingekoppelten Fremdenergie abgeleitet wird, ergibt sich der bereits oben erwähnte Vorteil, dass nämlich über die Fremdenergie das Retina-Implantat gesteuert werden kann.

Weiter ist es bevorzugt, wenn die Entladungsschaltung mit einer dritten Spannung verbunden ist, die aus der zweiten Spannung abgeleitet ist.

Hier ist von Vorteil, dass mit dem Abschalten der zweiten Spannung die dritte Spannung generiert wird, die die Entladungsschaltung aktiviert.

Auf diese Weise wird also beim Einschalten der zweiten Spannung der jeweilige Verstärker eingeschaltet und die positive Flanke des Spannungspulses generiert. Mit dem Ausschalten der zweiten Spannung wird die Entladungsschaltung aktiviert, was die negative Flanke des Spannungspulses liefert.

Insgesamt bietet diese Art der Steuerung des Retina-Implantates den Vorteil, dass über eine entsprechende Modulation der extern eingekoppelten Fremdenergie sämtliche Schaltvorgänge in dem Implantat gesteuert werden, Zeitglieder oder Frequenzgeneratoren sind im Implantat selbst für diese Zwecke nicht erforderlich.

Das insoweit beschriebene Implantat ist damit schaltungstechnisch sehr einfach aufgebaut, so dass eine hohe Dichte der einzelnen Elemente und damit auch der Stimulationselektroden erreicht werden kann. Diese hohe Dichte ermöglicht eine gute lokal aufgelöste Stimulation von Zellen in der Retina, wobei wegen der gepulsten Anregung und der jeweils vollständigen Rückführung der Elektrodenpolarisation eine effektive Stimulierung der Zellen möglich ist. Wegen der logarithmischen Kennlinie kann das neue Retina-Implantat darüber hinaus viele Zehnerpotenzen an Bildhelligkeit abdecken.

Es ist jedoch bevorzugt, wenn jedes Bildelement eine logarithmische Bildzelle für lokale Bildhelligkeit aufweist und jedem Bildelement zumindest eine logarithmische Bildzelle für globale Helligkeit zugeordnet ist, wobei vorzugsweise der Verstärker als Differenzverstärker ausgebildet ist, dessen einer Eingang mit der Bildzelle für lokale Bildhelligkeit und dessen anderer Eingang mit der Bildzelle für globale Helligkeit verbunden ist.

Diese Maßnahme ist aus der eingangs erwähnten DE 199 21 399 A1 an sich bekannt, wenn auch für Bildzellen mit linearer Kennlinie.

Wenn dieses "Differenzverstärkerprinzip", bei dem die Differenz zwischen der lokalen Bildhelligkeit und der über eine oder mehrere Referenzelemente genommenen globalen Helligkeit verstärkt und als Stimulationssignal weitergegeben wird, bei logarithmischen Bildzellen verwendet wird, so ergibt sich der besondere Vorteil, dass hier eine reine Kontrastverstärkung vorliegt, die mittlere Helligkeit eliminiert sich durch die Differenzbildung, denn sie ist im Logarithmus der Bildhelligkeit lediglich eine additive Größe.

Vor diesem Hintergrund betrifft die vorliegende Erfindung ebenfalls die Verwendung einer Bildzelle mit logarithmischer Kennlinie für ein Bildelement eines aktiven Retina-Implantates, das eine Vielzahl von Bildelementen aufweist, die einfallendes Licht in elektrische Stimulationssignale für über Stimulationselektroden zu kontaktierende Zellen der Retina umwandeln.

Weiter Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: eine schematische Darstellung des neuen Retina-Implantates in nicht maßstabsgetreuer Darstellung;
- Fig. 2: eine schematische Darstellung eines menschlichen Auges mit eingesetztem Retina-Implantat, ebenfalls nicht maßstabgetreu;
- Fig. 3: das Prinzipschaltbild der Spannungsversorgung für das Retina-Implantat aus Fig. 1;
- Fig. 4: die Ableitung der zweiten Spannung von dem extern eingekoppelten IR-Licht;
- Fig. 5: das prinzipielle Schaltbild einer Bildzelle für den Stimulationschip des Retina-Implantates aus Fig. 1;
- Fig. 6: die Spannungs- und Stromverläufe bei dem Bildelement aus Fig. 5;
- Fig. 7: eine detailliertere Darstellung des Aufbaus der Bildzelle aus Fig. 5; und
- Fig. 8: die Kennlinie der Bildzelle aus Fig. 7.

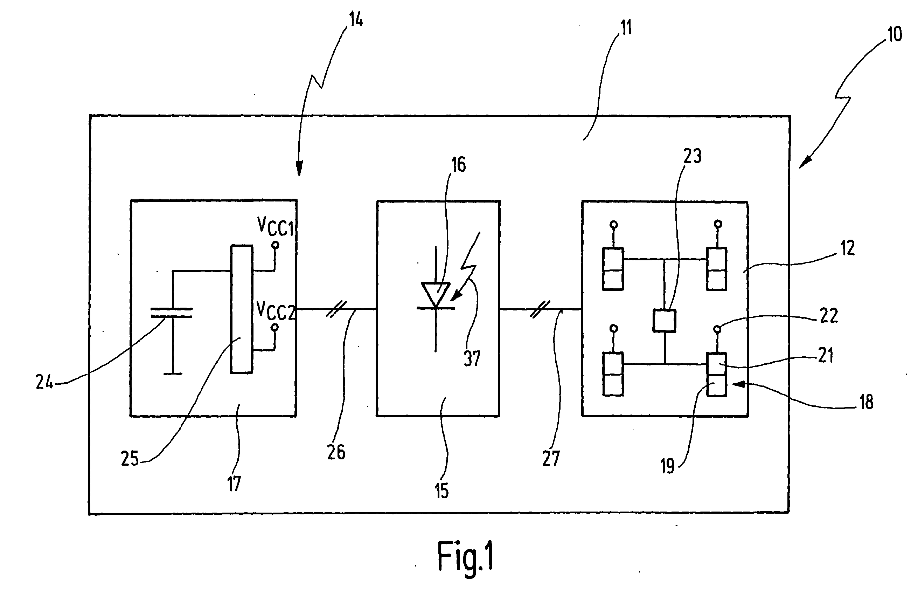

In Fig. 1 ist schematisch ein aktives Retina-Implantat 10 dargestellt, wobei die Abmaße nicht maßstabgetreu wiedergegeben sind.

Das Retina-Implantat 10 ist auf einer flexiblen Folie 11 ausgebildet, auf der ein Stimulationschip 12 sowie eine Energieversorgung 14 angeordnet sind. Die Energieversorgung 14 umfasst einen IR-Empfänger 15, der ein oder mehrere fotovoltaische Elemente 16 enthält, die auftreffendes IR-Licht in elektrische Spannung umwandeln. Die so eingekoppelte Fremdenergie wird an eine Spannungsversorgung 17 übergeben.

Der Stimulationschip 12 umfasst beispielsweise in Reihen und Spalten angeordnete Bildelemente 18, von denen in Fig. 1 der Übersichtlichkeit halber lediglich vier dargestellt sind. Jedes Bildelement 18 umfasst eine logarithmische Bildzelle 19 für lokale Bildhelligkeit sowie einen Verstärker 21, der an seinem Ausgang mit einer Stimulationselektrode 22 verbunden ist. Ferner ist auf dem Stimulationschip 12 eine Bildzelle 23 für globale Helligkeit vorgesehen, die mit den Verstärkern 21 sämtlicher Bildelemente 18 auf dem Stimulationschip 12 verbunden ist. Es versteht sich, dass der Stimulationschip 12 mehrere globale Bildzellen 23, oder aber auch nur eine einzige davon, umfassen kann.

Die Spannungsversorgung 17 weist ein Speicherelement 24 auf, in dem die von dem IR-Empfänger 15 aufgenommene Fremdenergie gespeichert wird. Das Speicherelement 24 ist mit einem Schaltungsteil 25 verbunden, das zwei verschiedene Spannungsversorgungen V_{cc1} und V_{cc2} in noch näher zu beschreibender Weise erzeugt. Über Leitungen 26 und 27 sind die Spannungsversorgung 17, der IR-Empfänger 15 und der Stimulationschip 12 miteinander verbunden.

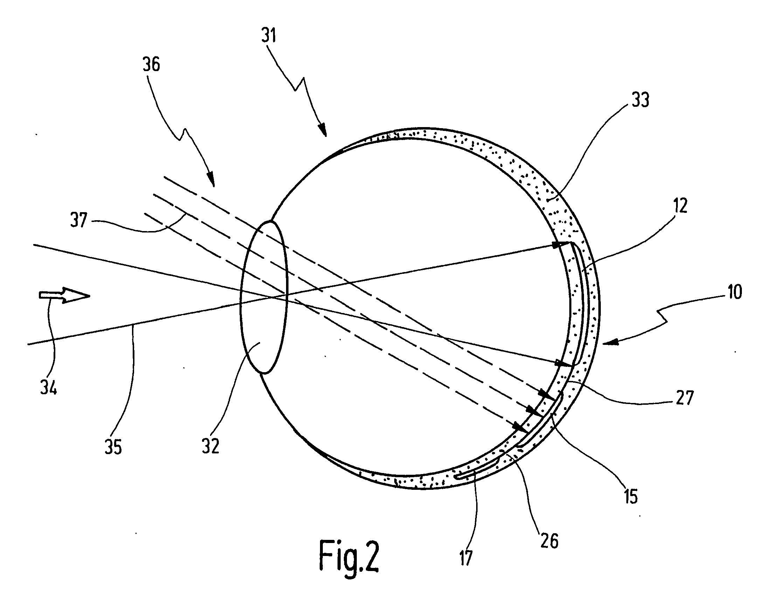

Das Retina-Implantat 10 aus Fig. 1 ist dazu bestimmt, in ein menschliches Auge 31 implantiert zu werden, das in Fig. 2 sehr schematisch dargestellt ist. Der Einfachheit halber sind nur die Linse 32 sowie die Retina 33 gezeigt, in die das Implantat 10 eingepflanzt wurde. Das Implantat 10 wird dabei vorzugsweise in den so genannten subretinalen Raum eingebracht, der sich zwischen dem Pigment-Epithel und der Fotorezeptorschicht bildet. Sofern die Fotorezeptorschicht degeneriert oder verloren ist, bildet sich der subretinale Raum zwischen dem Pigment-Epithel und der Schicht der Bipolar- und Horizontalzellen. Das Retina-Implantat 10 wird dabei so platziert, dass über die in Fig. 1 gezeigten Stimulationselektroden 22 Stimulationssignale auf Zellen in der Retina 33 ausgeübt werden können.

Durch einen Pfeil 34 angedeutetes sichtbares Licht, dessen Strahlengang bei 35 zu sehen ist, wird über die Linse 32 auf den Stimulationschip 12 geleitet, wo das sichtbare Licht 34 in elektrische Signale umgewandelt wird, die über die Verstärker 21 aus Fig. 1 in Stimulationssignale gewandelt werden.

In Fig. 2 ist zu erkennen, dass der IR-Empfänger 15 außerhalb des Einfallbereiches des sichtbaren Lichtes 34 liegt. Auf den IR-Empfänger 15 ist Fremdenergie 36 in Form von Strahlen von IR-Licht 37 gerichtet, das in dem IR-Empfänger in eine elektrische Spannung umgewandelt wird, die über die Leitungen 26 zunächst zu der Spannungsversorgung 17 gelangt, wo aus ihr entsprechende Versorgungsspannungen erzeugt werden. Diese Versorgungsspannungen gelangen dann über die Leitungen 26 und 27 zu dem Stimulationschip 12, wo sie verwendet werden, um das einfallende, sichtbare Licht 34 in noch näher zu beschreibender Weise in Stimulationssignale umzuwandeln.

Durch die räumliche Trennung von Stimulationschip 12 und IR-Empfänger 15 findet eine räumliche Entkopplung statt, so dass die unerwünschte Beeinträchtigung der Bildzellen im Stimulationschip 12 durch das IR-Licht 37 gering gehalten wird.

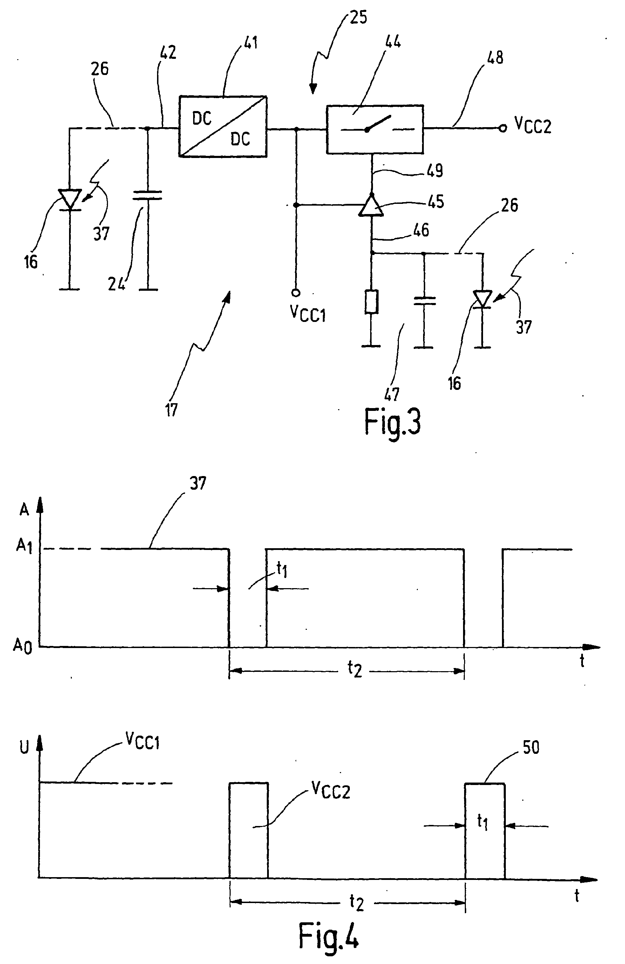

Wie aus dem IR-Licht 37 die erforderlichen Versorgungsspannungen generiert werden, wird jetzt an Hand von Fig. 3 beschrieben, wo die Spannungsversorgung 17 aus Fig. 1 detaillierter, aber dennoch schematisch dargestellt ist.

Die Spannungsversorgung 17 umfasst einen DC-DC-Wandler, der an seinem Eingang 42 mit dem Speicherelement 24 verbunden ist. Dieses Speicherelement 24 ist über die gestrichelt dargestellten Leitungen 26 mit dem fotovoltaischen Element 16 verbunden, das aus dem IR-Licht 37 eine elektrische Spannung generiert, die als Ladung in dem Speicherelement 24 gespeichert wird. Aus dieser Ladung, die eine Gleichspannung repräsentiert, erzeugt der DC-DC-Wandler 41 an seinem Ausgang 43 eine weitere Gleichspannung V_{cc1}, wie dies für DC-DC-Wandler an sich bekannt ist.

Der Ausgang 43 des DC-DC-Wandlers 41 ist ferner mit einem elektronischen Schalter 44 verbunden, der über einen Inverter 45 geschlossen und geöffnet wird. Der Inverter 45 ist an seinem Eingang mit einem RC-Glied 47 sowie über die gestrichelt dargestellten Leitungen 26 auch mit den fotovoltaischen Elementen 16 verbunden. An seinem Ausgang 48 gibt der elektronische Schalter 44 eine Spannung V_{cc2} ab, die gepulst ist, wie dies jetzt an Hand von Fig. 4 erläutert wird.

In Fig. 4 oben ist der zeitliche Verlauf des IR-Lichtes 37 gezeigt, das periodisch für die Zeitdauer t₁ von der Amplitude A1 auf die Amplitude A0 geschaltet wird. Diese "negativen Lichtpulse" der Dauer t₁ wiederholen sich in Zeitabständen t₂. Es versteht sich, dass das derart modulierte IR-Licht 37 während der Zeitdauer t₁ entweder ganz ausgeschaltet oder aber lediglich auf einen geringeren Intensitätswert abgesenkt werden kann.

Diese Modulation des IR-Lichtes 37 wird durch das Speicherelement 24 geglättet, so dass die Versorgungsspannung V_{cc1} konstant einen bestimmten Wert einnimmt, wie es in Fig. 4 unten gezeigt ist.

Über das RC-Glied 47 gelangt das modulierte IR-Licht 37 auf den Inverter 45, dessen Ausgang 49 auf L-Signal ist, solange das IR-Licht 37 auf Intensität A₁ ist. Während dieser Zeit ist der elektronische Schalter 44 geöffnet, so dass die Versorgungsspannung V_{cc2} auf 0 V liegt.

Während der Zeitspanne t₁ geht der Eingang 46 des Inverters 45 auf L-Signal, was bedeutet, dass sein Ausgang 49 auf H-Signal geht und der elektronische Schalter 44 schließt. Während der Zeitspanne t₁ geht damit die Versorgungsspannung V_{cc2} bspw. auf den selben Wert wie die Versorgungsspannung V_{cc1}. Der Ausgang 48 des elektronischen Schalters 44 liefert damit Spannungspulse 50 mit einer Pulslänge t₁ und einem Pulsabstand t₂.

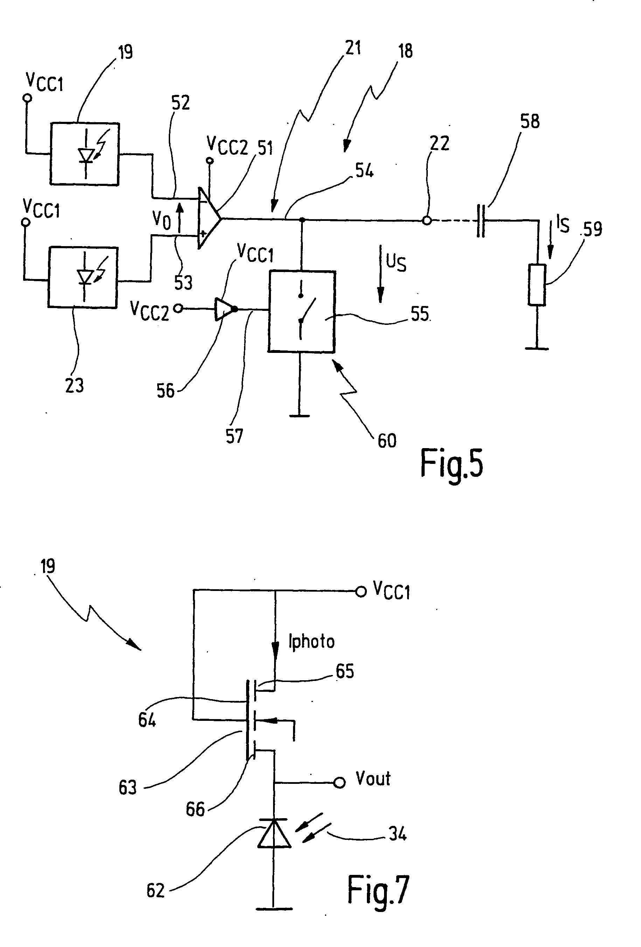

Wie diese Spannungspulse 50, die aus der Modulation des IR-Lichtes 37 abgeleitet werden, dazu eingesetzt werden, um den Stimulationschip 12 zu steuern, wird nun an Hand von Fig. 5 erläutert.

In Fig. 5 ist ein Bildelement 18 detaillierter, aber dennoch schematisch dargestellt.

Das Bildelement 18 umfasst einen Differenzverstärker 51, der an seinem invertierenden Eingang 52 mit der Bildzelle 19 für lokale Bildhelligkeit verbunden ist. An seinem nicht-invertierenden Eingang 53 ist der Differenzverstärker 51 mit der Bildzelle 23 für globale Helligkeit verbunden.

An seinem Ausgang 54 ist der Differenzverstärker 51 mit der Stimulationselektrode 22 verbunden. Ferner ist der Ausgang 54 mit einem elektronischen Schalter 55 verbunden, der über einen Inverter 56 angesteuert wird, der an seinem Ausgang als dritte Spannung 57 das invertierte Signal der Versorgungsspannung V_{cc2} liefert. Die Bildzellen 19 und 23 sowie der Inverter 56 werden über die Versorgungsspannung V_{cc1} mit Energie versorgt. Der Verstärker 51 dagegen wird über die Versorgungsspannung V_{cc2} mit Energie versorgt.

An den Eingängen 52, 53 des Differenzverstärkers 51 steht somit eine Differenzspannung V_{D} an, die die Differenz zwischen den Ausgangssignalen der Bildzellen 19 und 23 repräsentiert.

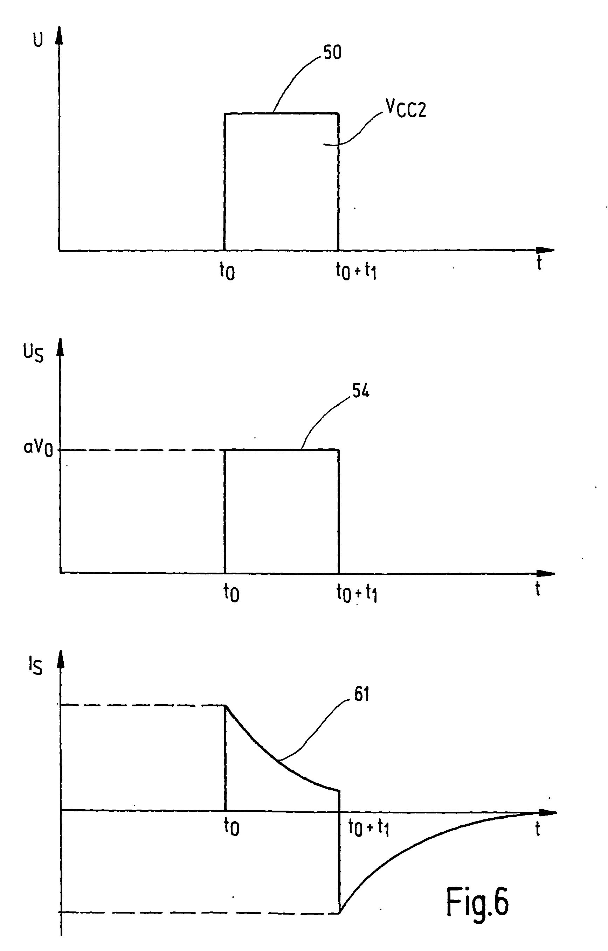

Der Differenzverstärker 51 selbst ist während der Zeitdauer t₂ jeweils nur für die Zeitdauer t₁ mit Energie versorgt, so dass er an seinem Ausgang 54 nur während der Ein-Zeit der Spannungspulse 50 aus Fig. 4 ein Stimulationssignal Uₛ abgibt. Dieser Zusammenhang ist in Fig. 6 gezeigt.

Am Ausgang 24 des Verstärkers 51 steht also während der Zeitspanne von t = t₀ bis t = t₀ + t₁ ein Spannungspuls an, dessen Amplitude aV_{D} der Intensität des auf die Bildzellen 19, 23 fallenden sichtbaren Lichtes entspricht.

Am Ende des Spannungspulses 50 geht der Inverter 56 an seinem Ausgang auf H-Signal und schließt den elektronischen Schalter 55, der auf diese Weise den Ausgang 54 mit elektrischer Masse verbindet.

Wie bereits eingangs erwähnt, bildet sich an der Stimulationselektrode 22 eine Helmholz-Doppelschicht, die für eine kapazitive Ankopplung der Stimulationselektrode 22 an das umgebende Gewebe in der Retina sorgt. In Fig. 5 ist diese kapazitive Ankopplung durch einen Kopplungskondensator 58 angedeutet, der über einen Widerstand 59, der das stimulierte Gewebe/die stimulierten Zellen repräsentiert, ebenfalls mit elektrischer Masse verbunden ist.

Zu Beginn des Spannungspulses 50 schaltet der Verstärker 51 an seinem Ausgang 54 auf die Spannung Uₛ = aV_{D}, was dazu führt, dass ein Stimulationsstrom Iₛ durch den Kopplungskondensator 58 in den Widerstand 59 fließt, wie dies in Fig. 6 unten dargestellt ist. Die Stärke des Stimulationsstromes Iₛ nimmt jetzt exponentiell ab, da sich der Kopplungskondensator 58 auflädt, was auch als Elektrodenpolarisation bezeichnet wird. Zum Zeitpunkt t = t₀ + t₁ schaltet der Spannungspuls 50 wieder ab, was bedeutet, dass die Versorgungsspannung V_{cc2} des Verstärkers 51 weggeschaltet wird. Gleichzeitig schaltet der elektronische Schalter 55 den Ausgang 54 auf die elektrische Masse, so dass der elektronische Schalter 55 als Entladungsschaltung 60 wirkt. Die in dem Kopplungskondensator 58 gespeicherte Ladung wird nun über den elektronischen Schalter 55 abgeführt, was sich durch einen negativen Stimulationsstrom Iₛ bemerkbar macht; siehe den Sprung im zeitlichen Verlauf 61 des Stimulationsstromes in Fig. 6 ganz unten. Der Kondensator 58 entlädt sich exponentiell, bis die Ladung in dem Kopplungskondensator 58 vollständig gelöscht wurde.

Es sei noch erwähnt, dass die Stärke des Stimulationsstromes Iₛ zum Einschaltzeitpunkt t = t₀ proportional zur Amplitude aV_{D} der Stimulationsspannung ist.

Durch das Ein- und Ausschalten der Versorgungsspannung V_{cc2}, was über die Modulation des eingekoppelten IR-Lichtes erreicht wird, wird also zunächst die positive Flanke der Stimulationsspannung Uₛ eingeschaltet, woraufhin ein Stimulationsstrom Iₛ fließt. Beim Ausschalten der Versorgungsspannung V_{cc2} fließt ein negativer Stimulationsstrom Iₛ, was zu einer Entladung des Kondensators 58 führt.

Die Pulslänge t₁ beträgt dabei 500 µs und der Pulsabstand t₂ 20 ms.

In Fig. 7 ist eine Bildzelle 19 detaillierter dargestellt. Jede Bildzelle 19 weist eine Fotodiode 62 auf, die in Sperrichtung betrieben wird. Die Bildzelle 19 weist ferner einen nMOS-Transistor 63 auf, dessen Gate-Elektrode 64 mit der Drain-Elektrode 65 zusammengeschaltet auf V_{cc1} liegt. An seiner Source-Elektrode 66 ist der nMOS-Transistor 63 mit der Kathode der Fotodiode 62 verbunden, deren Anode auf der elektronischen Masse liegt.

Durch die gezeigte Verschaltung wird der nMOS-Transistor 63 unterhalb des Schwellwertes betrieben, so dass der Spannungsabfall über dem nMOS-Transistor 63 exponentiell von dem Fotostrom Iₚₕₒₜₒ abhängt, dessen Stärke wiederum durch die Intensität des sichtbaren Lichtes 34 bestimmt wird, das auf die Fotodiode 62 fällt.

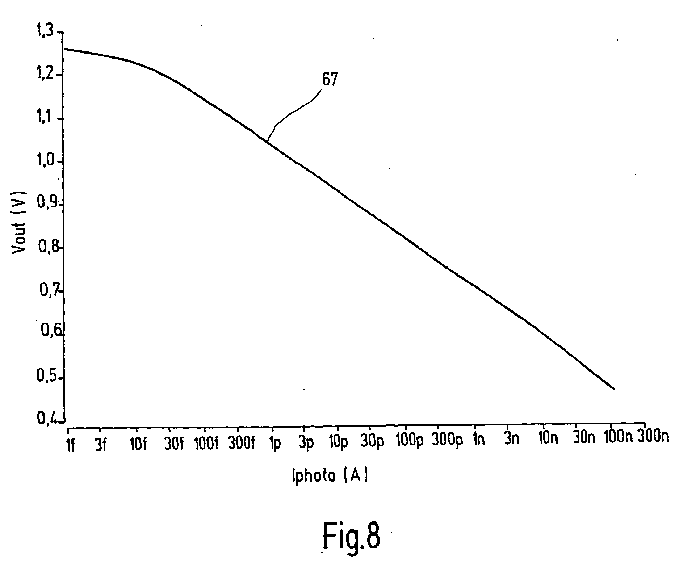

Diese Verschaltung führt dazu, dass die Ausgangsspannung Vₒᵤₜ der Bildzelle 19 aus Fig. 7 logarithmisch von der Intensität des Lichtes 34 abhängt, wie dies durch die Kennlinie 67 in Fig. 8 dargestellt ist.

Eine der Bildzelle 19 aus Fig. 7 vergleichbare Schaltung wird als Bildzelle 23 für globale Helligkeit verwendet.

Die Differenzspannung V_{D} in Fig. 5 repräsentiert damit die Differenz zwischen dem Logarithmus der lokalen und der globalen Bildhelligkeit. Da die globale Bildhelligkeit durch die mittlere Helligkeit bestimmt ist, die eine multiplikative Größe in der lokalen Helligkeit ist, lässt sich die mittlere Helligkeit durch die Differenz zwischen dem Logarithmus der lokalen und dem Logarithmus der globalen Helligkeit als additive Größe eliminieren.

Auf diese Weise verstärkt das jeweilige Bildelement 18 den Kontrast, so dass ein hoher Dynamikbereich gewährleistet ist und sich das Retina-Implantat an unterschiedliche Umgebungsbeleuchtungen anpassen kann.

## Patentansprüche

1. Aktives Retina-Implantat mit einer Vielzahl von Bildelementen (18), die einfallendes Licht (34) in elektrische Stimulationssignale (Uₛ) für über Stimulationselektroden (22) zu kontaktierende Zellen der Retina (33) umwandeln,
wobei jedes Bildelement (18)
zumindest eine Bildzelle (19), die einfallendes Licht (34) in elektrische Signale (Vₒᵤₜ) umwandelt, sowie
zumindest einen Verstärker (51) aufweist, dessen Eingang (52) mit der Bildzelle (19) und dessen Ausgang (54) mit zumindest einer Stimulationselektrode (22) verbunden ist, an die er ein Stimulationssignal (Uₛ) liefert,
und mit einer Energieversorgung (14), die extern eingekoppelte Fremdenergie (36) als Versorgungsspannung (V_{cc1}, V_{cc2}) für die Bildzellen (19) und die Verstärker (51) bereitstellt,
dadurch gekenntzeichnet, dass die Bildzelle (19) eine logarithmische Kennlinie (67) aufweist, nach der einfallendes Licht (34) bestimmter Intensität in elektrische Signale (Vₒᵤₜ) bestimmter Amplitude umgewandelt wird.

2. Retina-Implantat nach Anspruch 1, **dadurch gekennzeichnet; dass** das Stimu-Iationssignal (Uₛ) in Form von analogen Spannungspulsen bestimmter Pulslänge (t₁) und Pulsabstände (t₂) geliefert wird, deren Amplitude (aV_{D}) von der Intensität des einfallenden Lichtes (34) abhängt.

3. Retina-Implantat nach Anspruch 2, **dadurch gekennzeichnet, dass** der Ausgang (54) des Verstärkers (51) mit einer ansteuerbaren Entladungsschaltung (60) verbunden ist.

4. Retina-Implantat nach einem der Ansprüche 2 oder 3, **dadurch gekennzeichnet, dass** Pulslänge (t₁) und Pulsabstand (t₂) über die extern eingekoppelte Fremdenergie (36) bestimmt werden.

5. Retina-Implantat nach Anspruch 3, **dadurch gekennzeichnet, dass** die Entladungsschaltung (60) am Ende eines Spannungspulses derart angesteuert wird, dass der Ausgang (54) des Verstärkers (51) mit einem Entladepotenzial verbunden wird.

6. Retina-Implantat nach einem der Ansprüche 1 bis 5, **dadurch gekennzeichnet, dass** die Bildzellen (19) mit einer ersten Spannung (V_{cc1}) versorgt werden, die von einer zweiten Spannung (V_{cc2}) verschieden ist, mit der die Verstärker (51) versorgt werden.

7. Retina-Implantat nach Anspruch 6, **dadurch gekennzeichnet, dass** die zweite Spannung (V_{cc2}) mit der Pulslänge (t₁) und dem Pulsabstand (t₂) eingeschaltet wird:

8. Retina-Implantat nach einem der Ansprüche 6 oder 7, **dadurch gekennzeichnet, dass** die Entladungsschaltung (60) mit einer dritten Spannung (57) verbunden ist, die aus der zweiten Spannung (V_{cc2}) abgeleitet ist.

9. Retina-Implantat nach einem der Ansprüche 1 bis 8, **dadurch gekennzeichnet, dass** jedes Bildelement (18) eine Iogarithmische Bildzelle (19) für lokale Bildhelligkeit aufweist, und dass jedem Bildelement (18) zumindest eine logarithmische Bildzelle (23) für globale Helligkeit zugeordnet ist.

10. Retina-Implantat nach Anspruch 9, **dadurch gekennzeichnet, dass** der Verstärker (51) als Differenzverstärker ausgebildet ist, dessen einer Eingang (52) mit der Bildzelle (19) für lokale Bildhelligkeit und dessen anderer Eingang (53) mit der Bildzelle (23) für globale Helligkeit verbunden ist.

11. Verwendung einer Bildzelle (19) mit logarithmischer Kennlinie (67) für ein Bildelement (18) eines aktiven Retina-Implantates (10), das eine Vielzahl von Bildelementen (18) aufweist, die einfallendes Licht (34) in elektrische Stimulationssignale (Uₛ) für über Stimulationselektroden (22) zu kontaktierende Zellen der Retina (33) umwandeln.

## Claims

1. An active retina implant having a multiplicity of pixel elements (18) that convert incident light (34) into electric stimulation signals (Uₛ) for cells of the retina (33) with which stimulation electrodes (22) are to make contact,
wherein each pixel element (18) comprises
at least one image cell (19) that converts incident light (34) into electric signals (Vₒᵤₜ), and
at least one amplifier (51) whose input (52) is connected to the image cell (19) and whose output (54) is connected to at least one stimulation electrode (22) to which it supplies a stimulation signal (Us),
and having an energy supply (14) which provides externally coupled external energy (36) as supply voltage (V_{cc1}, V_{cc2}) for the image cells (19) and the amplifiers (51),
**characterized in that** the image cell (19) has a logarithmic characteristic (67) according to which incident light (34) of specific intensity is converted into electric signals (Vₒᵤₜ) of specific amplitude.

2. The retina implant of claim 1, **characterized in that** the stimulation signal (Uₛ) is supplied in the form of analog voltage pulses of specific pulse length (t₁) and pulse spacings (t₂, the pulse amplitude (aVₚ) being a function of the intensity of the incident light (34).

3. The active retina implant of claim 2, **characterized in that** the output (54) of the amplifier (51) is connected to a controllable discharge circuit (60).

4. The retina implant of anyone of claims 2 or 3, **characterized in that** pulse length (t₁) and pulse spacing (t₂) are determined via the externally coupled external energy (36).

5. The retina implant of claim 3, **characterized in that** the discharge circuit (60) is driven at the end of a voltage pulse in such a way that the output (54) of the amplifier (51) is connected to a discharging potential.

6. The retina implant of anyone of claims 1 to 5, **characterized in that** the image cells (19) are supplied with a first voltage (V_{cc1}) that differs from a second voltage (V_{cc2}) with which the amplifiers (51) are supplied.

7. The retina implant of claim 6, **characterized in that** the second voltage (V_{cc2}) is switched on in step with the pulse length (t₁) and the pulse spacing (t₂).

8. The retina implant of claim 6 or 7, **characterized in that** the discharge circuit (60) is connected to a third voltage (57) that is derived from the second voltage (V_{cc2}).

9. The retina implant of anyone of claims 1 to 8, **characterized in that** each pixel element (18) has a logarithmic image cell (19) for local image brightness, and **in that** each pixel element (18) is assigned at least one logarithmic image cell (23) for global brightness.

10. The retina implant of claim 9, **characterized in that** the amplifier (51) is designed as a differential amplifier of which one input (52) is connected to the image cell (19) for local image brightness, and of which the other input (53) is connected to the image cell (23) for global brightness.

11. Use of an image cell (19) having a logarithmic characteristic (67) for a pixel element (18) of an active retina implant (10) that has a multiplicity of pixel elements (18) that convert incident light (34) into electric stimulation signals (Uₛ) for cells of the retina (33) with which stimulation electrodes (22) are to make contact.

## Revendications

1. Implant rétinien actif avec une multitude d'éléments d'image (18), qui convertissent la lumière incidente (34) en signaux de stimulation électriques (Uₛ) pour les cellules de la rétine (33) à contacter à l'aide d'électrodes de stimulation (22),
sachant que chaque élément d'image (18)
présente au moins une cellule d'image (19), qui convertit la lumière incidente (34) en signaux électriques (Vₒᵤₜ), ainsi
qu'au moins un amplificateur (51), dont l'entrée (52) est reliée à la cellule d'image (19) et dont la sortie (54) l'est avec au moins une électrode de stimulation (22), à laquelle il fournit un signal de stimulation (Uₛ),
et avec une alimentation d'énergie (14), qui met à disposition de l'énergie externe (36) à couplage externe en tant que tension d'alimentation (V_{cc1}, V_{cc2}) pour les cellules d'image (19) et pour l'amplificateur (15),
**caractérisé en ce que** la cellule d'image (19) présente une ligne caractéristique logarithmique (67), conformément à laquelle la lumière incidente (34) d'une intensité donnée est convertie en signaux électriques (Vₒᵤₜ) d'une amplitude déterminée.

2. Implant rétinien selon la revendication 1, **caractérisé en ce que** le signal de stimulation (Uₛ) est fourni sous la forme d'impulsions de tension analogues de longueur d'impulsion (t₁) et d'intervalle d'impulsion (t₂)déterminés, dont l'amplitude (aV_{D}) dépend de l'intensité de la lumière incidente (34).

3. Implant rétinien selon la revendication 2, **caractérisé en ce que** la sortie (54) de l'amplificateur (51) est reliée à un circuit de décharge pouvant être commandé (60).

4. Implant rétinien selon l'une quelconque des revendications 2 ou 3, **caractérisé en ce que** la longueur d'impulsion (t₁) et l'intervalle d'impulsion (t₂) sont déterminés à l'aide de l'énergie externe (36) à couplage externe.

5. Implant rétinien selon la revendication 3, **caractérisé en ce que** le circuit de décharge (60) peut, à la fin d'une impulsion de tension, être commandé de telle que la sortie (54) de l'amplificateur (51) soit reliée à un potentiel de décharge.

6. Implant rétinien selon l'une quelconque des revendications 1 à 5, **caractérisé en ce que** les cellules d'image (19) sont alimentés à l'aide d'une première tension (V_{cc1}) , qui est différente d'une seconde tension (V_{cc2}), avec laquelle l'amplificateur (51) est alimenté.

7. Implant rétinien selon la revendication 6, **caractérisé en ce que** la seconde tension (V_{cc2}) est enclenchée avec la longueur d'impulsion (t₁) et l'intervalle d'impulsion (t₂).

8. Implant rétinien selon l'une quelconque des revendications 6 ou 7, **caractérisé en ce que** le circuit de décharge (60) est relié à une troisième tension (57), qui dérive de la seconde tension (V_{cc2})

9. Implant rétinien selon l'une quelconque des revendications 1 à 8, **caractérisé en ce que** chaque élément d'image (18) présente une cellule d'image logarithmique (19) pour la luminosité d'image locale, et **en ce qu'**à chaque élément d'image (18) est attribué au moins une cellule d'image logarithmique (23) pour la luminosité globale.

10. Implant rétinien selon la revendication 9, **caractérisé en ce que** l'amplificateur (51) est réalisé en tant qu'amplificateur différentiel, dont une entrée (52) est reliée à la cellule d'image (19) pour la luminosité locale et dont l'autre entrée (53) l'est avec la cellule d'image (23) pour la luminosité globale.

11. Utilisation d'un cellule d'image (19) à ligne caractéristique logarithmique (67) pour un élément d'image (18) d'un implant rétinien actif (10), qui présente une multitude d'éléments d'image (18), qui convertissent la lumière incidente (34) en signaux de stimulation électriques (Uₛ) pour les cellules de la rétine (33) à contacter à l'aide d'électrodes de stimulation (22).
